Europäisches Patentamt

European Patent Office

Office européen des brevets

(19)

(11) Veröffentlichungsnummer: **0 488 013 A1**

## (12) EUROPÄISCHE PATENTANMELDUNG

(21) Anmeldenummer: **91119657.4**

(22) Anmeldetag: **18.11.91**

(51) Int. Cl.5: **A61N 1/38**, A61N 1/39

(30) Priorität: **29.11.90 DE 4038105**

(43) Veröffentlichungstag der Anmeldung:
**03.06.92 Patentblatt 92/23**

(84) Benannte Vertragsstaaten:
**AT DE FR GB IT NL**

(71) Anmelder: **SIEMENS AKTIENGESELLSCHAFT**
**Wittelsbacherplatz 2**
**W-8000 München 2(DE)**

(72) Erfinder: **Kohl, Martin, Dipl.-Ing.**

**W-8521 Bräuningshof(DE)**

(54) **Defibrillator.**

(57) Bei einem Defibrillator (1), umfassend einen Speicherkondensator (2) und eine Spule (3), welche über biologisches Gewebe (4) zu einem RLC-Entladestromkreis (6) verbindbar sind, an dem eine Meßeinrichtung (9) über einen Transformator (10) angekoppelt ist, soll der Platzbedarf für die notwendigen Bauteile im RLC-Entladestromkreis (6) vermindert werden. Dazu ist die Spule (3) als Primärwicklung des Transformators (10) ausgebildet, wobei die Primärwicklung (3) die notwendige Induktivität des RLC-Entladestromkreises (6) aufweist.

FIG 1

EP 0 488 013 A1

Die vorliegende Erfindung bezieht sich auf einen Defibrillator, umfassend einen Speicherkondensator und eine Spule, welche über biologisches Gewebe zu einem LC-Entladestromkreis verbindbar sind, an dem eine Meßeinrichtung über einen Transformator angekoppelt ist.

Ein derartiger Defibrillator dient zur Beseitigung des Herzkammerflimmerns durch einen elektrischen Impuls (Elektroschock). Dazu wird elektrische Energie, die in einem Speicherkondensator (= Entladekondensator) gespeichert ist, über eine Induktionsspule in einer Entladeschaltung eines Entladekreises an biologisches Gewebe, z.B. Herzmuskelgewebe, eines Patienten abgegeben. Durch die Spule wird der gewünschte Verlauf (Kurvenform) des elektrischen Impulses im wesentlichen bestimmt. Um die abgegebene elektrische Energie und/oder den Widerstand des Patientengewebes bestimmen zu können, ist in einem mit dem Patienten über Elektroden verbindbaren Zweig des Entladestromkreises zusätzlich ein Transformator zur Auskopplung eines Meßsignales vorgesehen, der sekundärseitig mit einer Meßeinrichtung verbunden ist. Der Auskoppeltransformator ist als Stromtransformator ausgebildet. Die Meßeinrichtung mißt den an der Sekundärseite des Transformators auftretenden Spitzenwert. Aus dem gemessenen Spitzenwert werden der patientenwiderstand und/oder die an den Patienten abgegebene Energie unter Berücksichtigung der in dem Speicherkondensator gespeicherten Energie z.B. mittels eines Mikroprozessors bestimmt. Bei einem derartigen Defibrillator wirkt sich vor allem der Platzbedarf für die notwendigen Bauteile im Entladestromkreis nachteilig aus, insbesondere, wenn der Defibrillator in den Patienten implantierbar sein muß. Außerdem nimmt mit der Größe und Anzahl der Bauteile auch das Gewicht des Defibrillators zu.

Der Erfindung liegt die Aufgabe zugrunde, bei einem Defibrillator der eingangs genannten Art den Platzbedarf für die notwendigen Bauteile, insbesondere im Entladestromkreis, zu vermindern.

Diese Aufgabe wird durch einen Defibrillator nach Anspruch 1 gelöst.

Ein wesentlicher Vorteil der Erfindung besteht darin, daß der Platzbedarf für die notwendigen Bauteile im Entladestromkreis deutlich verringert ist. Das wird vor allem durch eine körperliche und funktionelle Kombination von zwei Einzelbauteilen - separate Induktionsspule und Primärwicklung des Auskoppeltransformators - zu einem Gesamtbauteil erreicht. Dazu wird die notwendige Induktivität im Entladestromkreis zur Erzeugung der gewünschten Impulsform des an den Patienten abgebbaren elektrischen Impulses allein durch die Primärwicklung des Auskoppeltransformators im Entladestromkreis gebildet. Durch eine Ausbildung des Transformators als kernloser Lufttransformator kann eine weitere Platzersparnis erreicht werden. Das führt gegenüber Transformatoren mit Eisenkern od.dgl. auch zu einer deutlichen Gewichtsersparnis.

Nach einer vorteilhaften Weiterbildung der Erfindung weist der Transformator eine sekundärseitige Induktivität auf, die klein gegenüber der primärseitigen Induktivität der Spule gewählt ist. Dadurch läßt sich das Gewicht und die Größe und damit auch der Platzbedarf des Transformators nochmals reduzieren. Die Primär- und Sekundärwicklung des Transformators können konzentrisch zueinander angeordnet werden. Dadurch erhält man eine kompakte, platzsparende Bauform, insbesondere, wenn die Primärwicklung als innere und die Sekundärwicklung als äußere Wicklung des Transformators ausgebildet sind.

Weitere Vorteile und Einzelheiten der Erfindung ergeben sich aus der nachfolgenden Beschreibung von Ausführungsbeispielen anhand der Zeichnungen und in Verbindung mit den Ansprüchen.

Es zeigen:

Figur 1 ein prinzipielles Blockschaltbild eines erfindungsgemäßen Defibrillators,

Figur 2 eine aperiodisch gedämpfte Schwingung, die als elektrischer Impuls an biologisches Gewebe abgebbar ist,

Figur 3 ein aus dem elektrischen Impuls gemäß Figur 2 differenziertes Signal an der Sekundärseite eines Transformators gemäß der Erfindung,

Figur 4 ein Teilschaltbild zu dem erfindungsgemäßen Defibrillator gemäß Figur 1 mit detaillierterer Darstellung einer Zeitmeßschaltung und

Figur 5 ein Rechtecksignal, das innerhalb der Zeitmeßschaltung gemäß Figur 4 erzeugt wird.

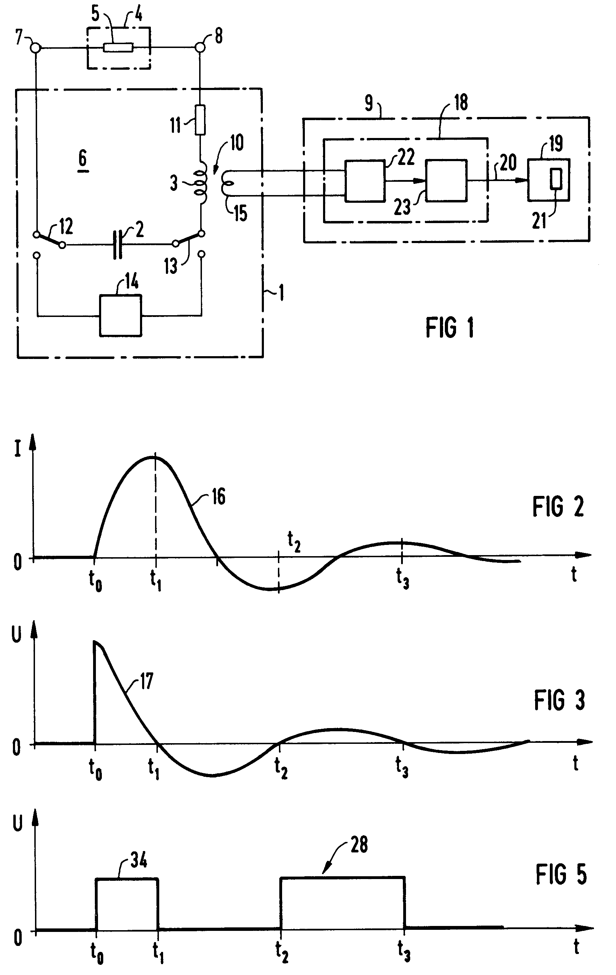

Figur 1 zeigt einen Defibrillator 1 mit einem Speicherkondensator 2 und einer Spule 3. Der Speicherkondensator 2 und die Spule 3 sind über biologisches Gewebe 4 eines Patienten, das einen Patientenwiderstand 5 aufweist, zu einem RLC-Entladestromkreis 6 verbindbar. Die Verbindung zum biologischen Gewebe 4 des Patienten kann bei insbesondere nicht im Patienten implantierten Defibrillatoren über an die Haut des Patienten ansetzbare Elektroden 7 und 8 geschehen. An den RLC-Entladestromkreis 6 ist eine Meßeinrichtung 9 über einen Transformator 10 angekoppelt. Dabei ist die Spule 3 als Primärwicklung des Transformators 10 ausgebildet. Diese Primärwicklung 3 weist die notwendige Induktivität des RLC-Entladestromkreises 6 auf, mit der die gewünschte Form des an den Patienten abgegebenen elektrischen Impulses im wesentlichen festgelegt wird.

Des weiteren ist noch ein Widerstand 11 im RLC-Entladestromkreis 6 dargestellt, der den Innenwiderstand der Spule 3, also den Innenwiderstand der Primärwicklung des Transformators 10, symbolisiert. Dieser Widerstand 11 und der Patientenwiderstand 5 bestimmen den Zeitpunkt, in welchem ein Strommaximum des an das biologische Gewebe 4 abgegebenen elektrischen Impulses erreicht wird.

Der Speicherkondensator 2 ist zum Aufladen über Schaltmittel 12 und 13, die auch elektronisch ausgebildet und gesteuert sein können, mit einer Auflade- und Steuerschaltung 14 verbindbar. Die Auflade- und Steuerschaltung 14 dient zum Laden des Speicherkondensators 2 und zum Steuern der Schaltmittel 12 und 13. Dadurch ist auch die Abgabe eines elektrischen Impulses aus dem RLC-Entladestromkreis 6 an das biologische Gewebe 4 steuerbar.

Der Transformator 10 ist in dem Ausführungsbeispiel gemäß Figur 1 als Spannungstransformator ausgebildet. Dadurch entfällt ein bei Stromtransformatoren erforderlicher Lastwiderstand an der Sekundärseite des Transformators. Das führt zu einer weiteren Platz- und Gewichtsersparnis. Eine weitere Verbesserung wird erreicht, wenn die Sekundärwicklung 15 des Transformators 10 eine Induktivität aufweist, die klein gegenüber der Induktivität der Primärwicklung 3 gewählt ist.

Die Abmessungen des Transformators 10 lassen sich auch dadurch verringern, indem der Transformators 10 im wesentlichen für Leerlaufbetrieb ausgebildet und hochohmig mit der Meßeinrichtung 9 verbunden ist. Eine weitere Gewichts- und Platzersparnis wird durch eine Ausbildung des Transformators als kernloser Lufttransformator erreicht. Durch die hochohmige Ankopplung des Transformators an die Meßeinrichtung 9 kann die Induktivität der Sekundärwicklung 15 des Transformators 10 bereits mit wenigen Windungen, z.B. zwei Windungen, realisiert werden. Dabei kann die Induktivität der Primärwicklung 3 beispielsweise 32 mH, die Kapazität des Speicherkondensators 2 ca. 32 $\mu$F und der Wert des Widerstandes 11 ca. 25 Ohm betragen.

Der beim erfindungsgemäßen Defibrillator vorgesehene Transformator bewirkt im dargestellten Ausführungsbeispiel eine Differenzierung eines in Figur 2 beispielhaft dargestellten und an das biologische Gewebe 4 abgebbaren elektrischen Impulses 16. An der Sekundärwicklung des Trafos 10 in Figur 1 bildet sich folglich ein differenziertes Signal 17 gemäß der Darstellung in Figur 3 aus. Sonach entspricht jeder Wendepunkt in der Kurve 16 zu den Zeitpunkten $t_1$, $t_2$ bzw. $t_3$ in Figur 2 jeweils einem Nulldurchgang der Kurve 17 zu den Zeitpunkten $t_1$, $t_2$ bzw. $t_3$ in Figur 3. Dieser Effekt der vorliegenden Erfindung wird ausgenutzt, indem die Meßeinrichtung 9 eine Zeitmeßschaltung 18 aufweist, mit der der zeitliche Abstand zwischen zwei vom Transformator übertragbaren elektrischen Werten des an das biologische Gewebe 4 abgebbaren elektrischen Impulses 16 meßbar ist. Solche Zeitmeßschaltungen lassen sich bei geringem Platzbedarf mit handelsüblichen Kleinbauteilen realisieren. Besonders genau und mit geringem Aufwand realisierbar ist dabei die Messung des zeitlichen Abstandes zwischen den Werten $t_0$ und $t_1$ der Kurve 17 (differenziertes Signal) gemäß Figur 3. Aus dem zeitlichen Abstand zwischen zwei charakteristischen Werten des elektrischen Impulses 16 ist sodann der Widerstand 5 des biologischen Gewebes 4 bestimmbar. Folglich ist bei geringem Platzbedarf zugleich eine hohe Meßgenauigkeit erreichbar.

Die an den RLC-Entladestromkreis 6 gemäß Figur 1 über den Transformator 10 angekoppelte Meßeinrichtung 9 weist eine Auswerteschaltung 19 auf. In der Auswerteschaltung 19 wird aus einem über die Leitung 20 aus der Zeitmeßschaltung 18 empfangenen Zeitsignal der Wert des Widerstandes 5 des biologischen Gewebes 4, z.B. mit Hilfe eines Mikroprozessors 21, bestimmt.

Die Zeitmeßschaltung 18 kann mehrere Baugruppen enthalten. Eine erste Baugruppe 22 dient zur Erkennung von charakteristischen ( = meßbaren) Werten des an das biologische Gewebe 4 abgebbaren elektrischen Impulses 16 bzw. im differenzierten Signal 17. In Abhängigkeit von solchen erkannten charakteristischen Werten kann die Baugruppe 22 dann eine Zählschaltung 23 steuern, die z.B. Taktimpulse zählt. So kann die Zählschaltung 23 z.B. durch die im Zeitpunkt $t_0$ entstehende Spannungsspitze des differenzierten Signales 17 gestartet und bei Vorliegen eines zweiten charakteristischen Wertes, z.B. Nulldurchgang zum Zeitpunkt $t_1$, gestoppt werden. Die Anzahl der gezählten Impulse stellt ein Maß ( = Zeitsignal) für den zeitlichen Abstand ( = Zeitdauer) zwischen beiden charakteristischen Werten dar.

Um die Meßeinrichtung 9 des Defibrillators 1 zu kalibrieren, können vom Hersteller zwischen den Elektroden 7 und 8 zunächst hochgenaue Meßwiderstände angeordnet werden und die bei Abgabe eines elektrischen Impulses 16 gemessenen Zeiten zwischen zwei charakteristischen Werten in der Auswerteschaltung 19, z.B. als Wertetabelle in Relation zum Wert des Meßwiderstandes in dem Mikroprozessor 21, abgelegt werden. Zwischenwerte können durch Interpolation errechnet und abgelegt werden. Es ist aber auch möglich, die jeweilige Zeitdauer bis zum Erreichen eines charakteristischen Wertes des Impulses 16 in Abhängigkeit von dem wechselnden Wert des Widerstandes 5 und von den elektrischen Werten der Primärwicklung 3, des Widerstandes 11 und des Kondensators 2 im Mikroprozessor 21 zu errechnen. Die mathematischen Grundlagen zum Verhalten eines RLC-Entladestromkreises sind z.B. aus der Zeitschrift "International Medical Device & Diagnostic Industry" Mai/Juni 1990, Seite 48, bekannt.

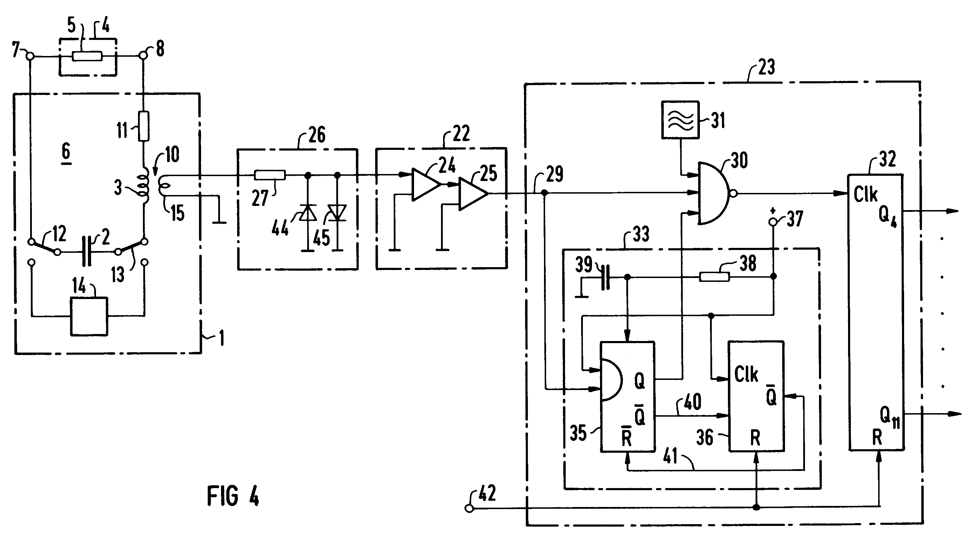

In Figur 4 sind die Zählschaltung 23 und die erste Baugruppe 22 der zu Figur 1 beschriebenen Zeitmeßschaltung 18 als ein spezielles Ausführungsbeispiel dargestellt. Die Baugruppe 22 enthält zwei Komparatorschaltungen 24 und 25, die über eine Begrenzungsschaltung 26 mit dem zu Figur 1 beschriebenen RLC-Entladestromkreis 6 in dem Defibrillator 1 verbunden sind. Die Begrenzerschaltung 26 enthält einen Begrenzungswiderstand 27 und zwei antiparallel geschaltete Dioden 44 und 45. Mit der nicht zwingend erforderlichen Begrenzerschaltung 26 wird die in Figur 3 auf den Zeitpunkt $t_0$ folgende Spannungsspitze des differenzierten Signales 17 begrenzt. Dadurch wird der Eingang der nachfolgenden Komparatorschaltung 24 vor Überspannungen geschützt. Der Ausgang des Komparators 24 ist mit einem Eingang des Komparators 25 verbunden. Sonach wird jeder Nulldurchgang des differenzierten Signals gemäß Kurve 17 in Figur 3 besonders genau bestimmt.

Durch die spezielle Ausbildung der Baugruppe 22 gemäß Figur 4 entsteht an deren Ausgang ein Rechtecksignal 28, das in Figur 5 dargestellt ist. Mit diesem Rechtecksignal 28 wird über eine Leitung 29 eine Torschaltung in der Zählschaltung 23 angesteuert (Figur 4). Über einen anderen Eingang erhält die Torschaltung 30 aus einem Taktgenerator 31 Taktimpulse. Die Taktimpulse werden in einem Zählerbauteil 32 während der Zeitdauer gezählt, während der die Torschaltung 30 z.B. durch das Rechtecksignal 28 (insbesondere durch den Einzelimpuls 34) auf der Leitung 29 geöffnet worden ist. Als Zählerbauteil eignet sich z.B. ein 11-Bit-Zähler-IC mit der Typenbezeichnung 4020, wobei dieser integrierte Schaltkreis 4020 Parallelausgänge $Q_4$ bis $Q_{11}$ aufweist. Das elektrische Ausgangssignal aus dem Zählerbauteil 32 kann dann über eine entsprechend vielpolig ausgebildete Leitung 20 der Auswerteschaltung 19 zur Bestimmung eines Parameters zugeführt werden, wie es zu der Figur 1 bereits erwähnt ist.

Wenn der an das biologische Gewebe 4 abgebbare elektrische Impuls die Form einer gedämpften Schwingung gemäß Kurve 16 in Figur 2 haben kann, ist es zweckmäßig, eine Zusatzschaltung 33 gemäß Figur 4 vorzusehen. Mit dieser Zusatzschaltung 33 wird die Torschaltung 30 zusätzlich derart gesteuert, daß nur während der Zeitdauer eines ersten Impulses 34 ($t_0$ bis $t_1$) des Rechtecksignales 28 gemäß Figur 5 Zählimpulse aus dem Taktgenerator 31 gemäß Figur 4 zu dem Zählerbauteil 32 gelangen können. Dazu weist die Zusatzschaltung 33 zwei Kippstufen auf. Eine Kippstufe ist als monostabiler Multivibrator 35 und eine andere Kippstufe als bistabiler Multivibrator 36 ausgebildet. Die monostabile Kippstufe 35 erhält über den Anschluß 37 eine positive Betriebsspannung. Die positive Betriebsspannung speist auch ein RC-Glied, das aus einem Widerstand 38 und einem Kondensator 39 gebildet ist. Die monostabile Kippstufe 35 wird durch die erste Flanke im Zeitpunkt $t_0$ des Impulses 34 des Rechtecksignales 28 gestartet, das auf der Leitung 29 bei Abgabe eines Impulses 16 an das biologische Gewebe 4 entsteht. Dadurch wird an einem Ausgang Q der Kippstufe 35 ein Ausgangssignal erzeugt, mit dem die Torschaltung 30 während der durch das RC-Glied 38, 39 eingestellten Standzeit der Kippstufe 35 geöffnet ist. Ein invertierender Ausgang $\overline{Q}$ der Kippstufe 35 ist über eine Leitung 40 mit einem Steuereingang CLK der bistabilen Kippstufe 36 verbunden. Die bistabile Kippstufe 36 sperrt sodann über eine Leitung 41 die monostabile Kippstufe 35, so daß diese von eventuell nachfolgenden Impulsen des Rechtecksignales 28 nicht mehr auslösbar ist. Vor der Abgabe eines weiteren elektrischen Impulses 16 an das biologische Gewebe 4 kann die Zusatzschaltung 33 und der Zähler 32 durch ein über den Anschluß 42 eingebbares Resetsignal, z.B. aus der Auflade- und Steuerschaltung 14, wieder in den Ausgangszustand zurückgesetzt werden. Als monostabile Kippstufe 35 kann z.B. ein Baustein mit der Typenbezeichnung 4538 und als bistabile Kippstufe ein Baustein mit der Typenbezeichnung 4013 verwendet werden.

**Patentansprüche**

1. Defibrillator, umfassend einen Speicherkondensator (2) und eine Spule (3), welche über biologisches Gewebe (4) zu einem RLC-Entladestromkreis (6) verbindbar sind, an dem eine Meßeinrichtung (9) über einen Transformator (10) angekoppelt ist, wobei die Spule (3) als Primärwicklung des Transformators (10) ausgebildet ist und die Primärwicklung (3) die notwendige Induktivität des RLC-Entladestromkreises (6) aufweist.

2. Defibrillator nach Anspruch 1, wobei der Transformator (10) kernlos als Lufttransformator ausgebildet ist.

3. Defibrillator nach Anspruch 1 oder 2, wobei der Transformator (10) sekundärseitig eine Induktivität aufweist, die klein gegenüber der Induktivität der Primärwicklung (3) gewählt ist.

4. Defibrillator nach einem der Ansprüche 1 bis 3, wobei der Transformator (10) im wesentlichen für Leerlaufbetrieb ausgebildet und hochohmig mit der Meßeinrichtung (9) verbunden ist.

5. Defibrillator nach einem der Ansprüche 1 bis 4, wobei der Transformator (10) als Spannungstransformator ausgebildet ist.

**6.** Defibrillator nach einem der Ansprüche 1 bis 5, wobei die Primärwicklung (3) und die Sekundärwicklung (15) des Transformators (10) konzentrisch zueinander angeordnet sind.

**7.** Defibrillator nach Anspruch 6, wobei die Primärwicklung (3) als innere und die Sekundärwicklung (15) als äußere Wicklung der konzentrisch zueinander angeordneten Wicklungen (3, 15) des Transformators (10) ausgebildet sind.

**8.** Defibrillator nach einem der Ansprüche 1 bis 7, wobei die Meßeinrichtung (9) eine Zeitmeßschaltung (18) aufweist, mit der der zeitliche Abstand zwischen zwei vom Transformator (10) übertragenen elektrischen Werten ($t_0$ bis $t_3$) eines an das biologische Gewebe (4) abgebbaren elektrischen Impulses (16) meßbar ist.

**9.** Defibrillator nach Anspruch 8, wobei mit der Meßeinrichtung (9) aus dem zeitlichen Abstand zwischen zwei charakteristischen Werten ($t_0$ bis $t_3$) des elektrischen Impulses (16) der Wert des Widerstandes (5) des biologischen Gewebes (4) bestimmbar ist.

FIG 1

FIG 2

FIG 3

FIG 5

FIG 4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | EP-A-0 060 404 (HUBER) <br> * Seite 3, Zeile 1 - Seite 4, Zeile 29; Abbildungen 1,2 * | 1 | A61N1/38 <br> A61N1/39 |
| A | | 5 | |
| | --- | | |
| A | EP-A-0 315 368 (CHARBONNIER,BENVEGAR,ROCKWELL) <br> * Spalte 3, Zeile 50 - Spalte 6, Zeile 33; Abbildungen 1,2 * | 1,8,9 | |
| | --- | | |
| A | GB-A-2 085 593 (CHARBONNIER,LONG) | 1,8,9 | |
| | --- | | |
| A | US-A-4 771 781 (LERMAN) | 1,8,9 | |
| | ----- | | |
| | | | **RECHERCHIERTE SACHGEBIETE (Int. Cl.5)** |
| | | | A61N |

Der vorliegende Recherchenbericht wurde für alle Patentansprüche erstellt

| Recherchenort | Abschlußdatum der Recherche | Prüfer |
|---|---|---|
| DEN HAAG | 20 MAERZ 1992 | HERBELET J.C. |